# EUROPÄISCHE PATENTANMELDUNG

(11) **EP 2 745 862 A2**
(43) Veröffentlichungstag der Anmeldung: **25.06.2014**
(21) Anmeldenummer: 14000710.5
(22) Anmeldetag: 15.12.2011
(51) Int. Cl.: A61M 1/16

(54) **Mischanlage**

(30) Priorität: 23.12.2010 DE 102010055781
(62) Teilanmeldung aus: 11009861.3
(71) Anmelder: Völker, Manfred, 63825 Blankenbach (DE)
(72) Erfinder: Völker, Manfred, 63825 Blankenbach (DE)
(74) Vertreter: Flosdorff, Jürgen

(57) **Zusammenfassung**

Beutelporter für Fertiglösung mit zwei isolierten Schalen (59a, 59b), die auseinander klappbar sind und einen oberen Schlitz (71) aufweisen, durch den ein leerer Beutel (54) einschiebbar ist, der einen Zulaufanschluss (43) aufweist, durch den die Fertiglösung in den Beutel (54) einfüllbar ist, wonach sich der Beutel (54) an die Innenkonturen der Schalen (59a, 59b) anlegt, die mit innen liegenden Heizmatten (60a, 60b) versehen sind, die einen Temperaturverlust ausgleichen können,
wobei der Beutel (54) eine Anschlussleitung (46) mit einem Überleitungssystem (57) zum Patienten aufweist.

## Beschreibung

Die Erfindung betrifft eine Mischanlage zur Herstellung gebrauchsfähiger Lösungen aus flüssigen, pulvrigen, pastösen oder granulierten Konzentraten und einer hochreinen Flüssigkeit für den Einsatz als Konzentrat oder Spüllösung in der Medizintechnik, insbesondere für die Hämodialyse. Weitere Anwendungsgebiete für die mit der Mischanlage hergestellten Lösungen sind Purisolelösungen für den Einsatz während einer Blasenoperation oder einer postoperativen Blasenspülung, Peritoneallösungen für eine Bauchfell-Dialysebehandlung, Spüllösungen für chirurgische Eingriffe und Spüllösungen für Athroskopie, wobei diese Aufzählung nicht abschließend zu verstehen ist.

Die Mischanlage enthält eine Reinstwasserquelle, z.B. eine Umkehrosmose, die bevorzugt unter Verwendung eines Sterilfilters das von ihr erzeugte Permeat zu der Reinstwasserzulaufleitung des Mischgerätes führt, wobei das Mischgerät einen Rechner und einen Rezirkulationskreislauf enthält, in den eine Pumpe, eine Heizeinrichtung, ein Rohstoffbehälter, der vor Beginn des Mischvorgangs flüssiges, pastöses, pulvriges oder granuliertes Konzentrat enthält, und ein Fertiglösungsbehälter eingeschaltet sind, der vor Beginn des Mischvorgangs mit einem definierten Volumen des Reinstwasser befüllbar ist.

Es ist seit langem bekannt, gebrauchsfertige Lösungen zentral herzustellen und zu dem Verbraucher zu versenden, wobei dies mit hohen Frachtkosten verbunden ist. Daneben sind dezentrale Mischanlagen bekannt, die in vielerlei Hinsicht Nachteile aufweisen. Sie sind unzureichend hinsichtlich der Produktgenauigkeit. Beispielsweise sind bei Dialysekonzentraten Natriumabweichungen von maximal +/- 2,5% erlaubt. Ferner sind sie hinsichtlich der Hygieneanforderungen unzureichend, wobei die Anforderungen bei der Mikrobiologie < 100 kBE und < 0,25 EU lauten. Die chemischen und thermischen Desinfektionsmaßnahmen sind teuer und aufwändig. Außerdem sind dezentrale Mischanlagen groß und auf fixe Tanks bzw. Behälter beschränkt.

Es kommt hinzu, dass die Verteilung bzw. Anwendung am Benutzungsort teuer bzw. aufwändig ist. Nach erfolgter Mischung ist die Lösung kalt und damit als Spüllösung nicht gebrauchsfertig.

Der vorliegenden Erfindung liegt die Aufgabe zugrunde, wenigstens einige der oben genannten Nachteile des Standes der Technik zu vermeiden. Insbesondere besteht eine Aufgabe darin, eine Mischanlage anzugeben, bei der unter Verwendung von hochreiner, keimfreier Flüssigkeit eine hochgenaue Zudosierung der Flüssigkeit gewährleistet ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass in der Zulaufleitung der Reinstwasserquelle oder in der Zulaufleitung des Mischgerätes wenigstens ein mit einem zugehörigen Rechner verbundener Flussmesser eingeschaltet ist, und dass stromabwärts davon eine volumetrische Messkammer angeordnet ist, in der der Füllstand der Messkammer ermittelbar ist, wobei der ermittelte Wert durch den Rechner des Mischgerätes mit dem Messwert des wenigstens einen Flussmessers verglichen und dessen Messwert erforderlichenfalls korrigiert wird.

Erst wenn die Angabe des wenigstens einen Flussmessers über die Durchflussmenge mit dem bekannten Messwert der Messkammer in Einklang steht und der Flussmesser kalibriert ist, wird die für den jeweiligen Mischvorgang vorgesehene Reinstwassermenge unter Berücksichtigung der zum Zwecke der Kalibrierung bereits zugelaufenen Wassermenge in den Rezirkulationskreislauf eingefüllt, so dass diese Zudosierung mit höchster Genauigkeit erfolgt.

Dabei ist bevorzugt, dass zwei Flussmesser hintereinander angeordnet sind, die als Betriebsflussmesser und Überwachungsflussmesser redundant arbeiten und jeweils von zugehörigen Rechnern überwacht bzw. kalibriert werden. Hierdurch wird die hochgenaue Zudosierung im höchsten Maße ausfallsicher.

Die Zulaufleitung des Mischgerätes führt zu einem Füllventil des Zirkulationskreislaufs. Die Messkammer kann grundsätzlich in Strömungsrichtung vor dem Füllventil angeordnet sein, es ist jedoch bevorzugt, dass die Messkammer in den Rezirkulationskreislauf eingeschaltet ist. Dies ist insbesondere dann der Fall, wenn die Messkammer so ausgestattet ist, dass sie nicht nur den Füllstand erfasst (d.h. den Zeitpunkt des Erreichens eines festliegendes Füllniveaus in der Messkammer erfasst, wobei das Volumen der zuführenden Leitung und der Messkammer bekannt sind), sondern auch derart, dass in der Messkammer die Dichte der durchströmenden Flüssigkeit erfasst werden kann. Hierzu kann an der Außenwand der Messkammer ein Ultraschalldichtesensor mit seinen zugehörigen Komponenten angeordnet sein.

In einer bevorzugten Ausgestaltung der Erfindung hat die Messkammer einen oberen transparenten Abschnitt, der mit optoelektronischen Mitteln versehen ist, mit der der Füllstand, bzw. das Erreichen einer bestimmten Füllhöhe, messbar ist.

Alternativ hierzu kann in die Messkammer ein kapazitiver Füllstandssensor eingesetzt sein.

Außerdem wird vorgeschlagen, dass die Messkammer mit einer elektrolytischen Ozonzelle versehen sein kann, wobei es sich um eine kostengünstige Desinfektionsmaßnahme handelt.

Das Mischgerät enthält ferner an geeigneten Stellen Mittel zur Überwachung der Leitfähigkeit der hergestellten Flüssigkeit und ihrer Temperatur. An den Rezirkulationskreislauf ist ein Transferventil angeschlossen, so dass die gebrauchsfähig erzeugte Lösung zu Spülzwecken direkt einsetzbar ist oder in weitere Lagerbehälter abgefüllt wird.

Der Messzylinder hat ein bekanntes Volumen (ebenso wie die zuführende Leitung) und einen sich nach oben verjüngenden zylindrischen Endabschnitt, der bevorzugt transparent ist. An diesem transparenten Ende kann elektronisch der Füllstand ermittelt und über den Rechner der Mischanlage mit den Flussmesserwerten verglichen werden.

Das Gerät ermöglicht die Online-Überprüfung der Lösung mittels Dichte-, Leitfähigkeits- und Temperaturmessungen während jedes Mischvorgangs. Die erzeugte Lösung, die auf einer vorgegebenen Temperatur gehalten wird, lässt sich auf einfache Weise verteilen.

Nach einem weiteren Vorschlag der Erfindung ist der Rezirkulationskreislauf mit wenigstens einem Druckluftanschluss versehen, um die fertig gemischte Lösung mittels der Druckluftzuführung zu fördern und zu entleeren.

Als Rohstoffbehälter können Wegwerfbehälter oder auch wiederbefüllbare Behälter verwendet werden. Nach einem besonders vorteilhaften Vorschlag enthält der Rohstoffbehälter einen unteren tangentialen Zulauf und einen oberen tangentialen Ablauf, wobei außerdem ein zusätzlicher unterer tangentialer Ablauf kleineren Durchmessers vorgesehen ist, durch den Flüssigkeit aus dem Behälter über eine außerhalb des Behälters angeordnete Leitung in die obere Ablaufleitung eingeführt wird, wenn der Behälterinhalt so verklumpt und verstopft ist, dass (zunächst) kein Ablauf durch die obere tangentiale Auslauföffnung erfolgen kann.

Weiter kann vorgesehen sein, dass der Rohstoffbehälter einen verschließbaren Deckel mit einem nach innen ragenden, mittigen, kegelförmigen Ansatz aufweist, der den Freiraum in dem Behälter während des Mischvorgangs ausfüllt. Dies führt zu einer sehr intensiven Vermischung in dem Behälter, wobei sich infolge des kegelförmigen Deckels weniger Feststoffe an der Wand des Behälters ablagern.

Als Fertiglösungsbehälter (die zunächst mit Reinstwasser befüllt werden) können mobile oder fixe Tanks bzw. Beutel verwendet werden. Der Tank bzw. Beutel kann über die tiefste Stelle befüllt und entleert werden. Es kann aber auch vorgesehen sein, dass die Flüssigkeitszirkulation über die höchste Stelle des Tanks zugeführt und über die niedrigste Stelle des Tanks wieder in den Zirkulationskreislauf eintritt.

Bevorzugt ist ferner, dass bei der Verbindung einer Umkehrosmose mit dem Mischgerät zu einer Einheit eine gemeinsame Steuerung vorgesehen ist.

Weitere Merkmale und Einzelheiten der Erfindung ergeben sich aus der nachfolgenden Beschreibung sowie anhand der beigefügten Figuren.

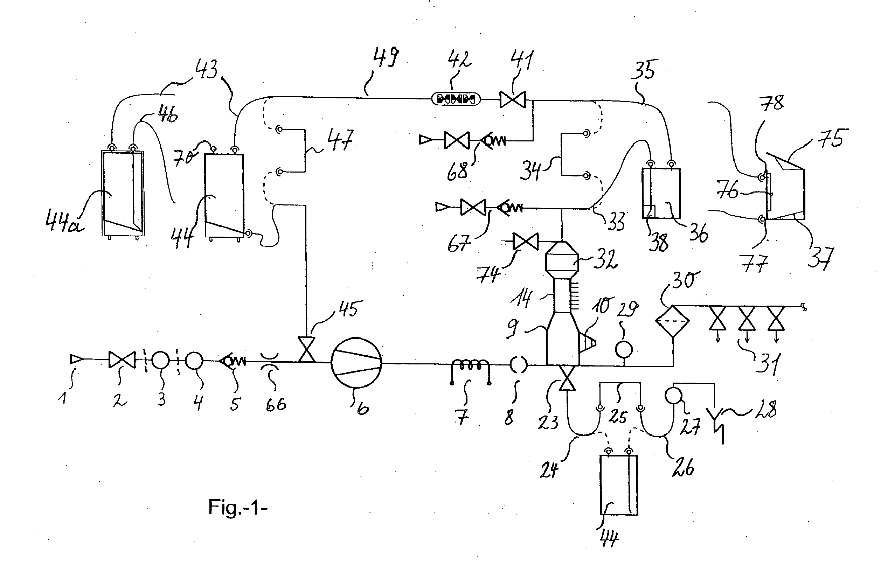

Figur 1 zeigt den Flüssigkeitsverlauf über die Reinstwasserzulaufleitung (1), das Wassereingangsventil (2), die Flussmesser (3, 4) mit den Beruhigungsstrecken (65), zur Verbesserung der Flussmessergenauigkeit, das Rückschlagventil (5) und die wahlweise einsetzbare Konstantflussdrossel (66), die zur Erhöhung der Flussmessergenauigkeit beiträgt. Zur Validierung der Flussmesser (3,4) wird Flüssigkeit bei geöffneten Wassereingangsventil (2) über Pumpe (6), Heizer (7), Leitfähigkeitsmessung (8) in die zuvor entleerte Messkammer (9) tangential gefördert. Die Leitfähigkeits-messung (8) kann in beliebigen Stellen des Zirkulationskreislaufes angeordnet sein. Bei bekanntem Volumen der Messkammer (9) wird die Flüssigkeit entweder mittels Optoelektronik in der transparenten Messtrecke (14) ermittelt und durch den Rechner der Mischanlage (hier nicht dargestellt) mit den Flussmesserergebnissen (3/4) verglichen. Dabei ist die Messkammer (9) nicht auf den gezeichneten Einbauort beschränkt, sondern kann mit Vorteil auch stromaufwärts des Zirkulations- und Füllventiles (45) eingebaut werden. Ebenso besteht neben der optoelektronischen Messung auch die Möglichkeit kapazitiv oder über einen Näherungsschalter den Füllstand abzufragen. Alternativ zur Messkammer (9) kann bei bekannter Tank- oder Beutelgeometrie die Validierung der Flussmeser (3/4) auch mittels eines Füllstandssensores im unteren Bereich des Tanks (44) oder des Folienbeutels (54) im Behälter (59) verwendet werden. Der untere Bereich des Tanks bzw. Beutels wird gewählt, weil bereits bei geringen zugeführten Volumina ein Aussage zur Genauigkeit der Flussmesser (3,4) zu treffen ist.

Bei Übereinstimmung der Messwerte der optoelektronischen Messung und der Flussmesser wird Ventil (45) geöffnet und der Tank bzw. das Behältnis (44/44a) mit der voreingestellten und durch die Flussmesser (3,4) überwachten Menge gefüllt. Dabei arbeiten Flussmesser (3) als Betriebsflussmesser und Flussmesser (4) als Überwachungsflussmesser redundant. Der Rechner der Mischanlage enthält einen Betriebs- und einen Schutzsystemrechner. Diese werten die Flussmesserergebnisse (3/4) unabhängig voneinander aus und vergleichen die Ergebnisse während des gesamten Füllprozesses. Die Flussmesser (3,4) können dabei auch in einer vorgeschalteten Reinstwasseranlage eingebaut sein, die mit der Mischeinheit eine Einheit bildet.

Während des Füllvorganges bleibt Ventil (41) geschlossen, so dass keine weitere Flüssigkeit über die Messkammer (9) und den angeschlosssenen Rohstoffbehälter (37,38) fließt.

Vorzugsweise ist das Behältnis (44) ein stationärer Tank mit Tankentlüftung (70) und Behältnis (44a) vorzugsweise ein Behältnis mit Folienbeutel (54). Bei Verwendung eines stationären Tankes (44) besteht die Möglichkeit, den Teil der Zirkulationsstrecke (43) mit einem Sprühkopf abzuschließen.

Nach abgeschlossener Füllung des Behälters (44) wird die Pumpe (6) eingeschaltet und Permeat über die Rohstoffbehälter (36/37) bzw. die angeschlossenen Leitungen (33/35) zirkuliert. Dabei wird das Ventil (41) geöffnet und die Lösung über den statischen Mischer (42) so vermischt, dass zwei sich kreuzende Teilströme entstehen. Vorzugsweise besteht der statische Mischer aus zwei symentrisch gedrehten Profilen, die in ein Rohr oder wahlweise im gesamten Zirkulationskreis eingebaut sein können. Die so zirkulierte Flüssigkeit wird über die Zulaufleitung (49) zum Fertiglösungsbehälter (44) zirkuliert. Während der Zirkulation wird je nach Erfordernis die Heizung (7) zugeschaltet und Temperatur- und Leitfähigkeit über Sensor (8) überwacht. Wobei Heizer (7) auch in der vorgeschalteten Reinstwasseranlage eingebaut werden kann. In diesem Fall wird die Mischanlage mit bereits vorgewärmtem Reinstwasser gefüllt.

Ebenso ist eine Überwachung der Dichte über eine Ultraschallmessung, eine Kombination aus Druck und Füllstand, oder eine annähernde Dichtemessung über einen kapazitiven Füllstandssensor möglich. Es besteht auch die Möglichkeit, über ein im Zirkulationskreis eingebautes Probeentnahmeventil (79) (nicht dargestellt) eine manuelle Probe für Laboruntersuchung zu entnehmen. Denkbar sind auch Qualitätsnachweise über Ionensensitive Onlinemessungen.

Die Pumpe (6) kann dabei über Druckaufnehmer (29) so gesteuert bzw. geregelt werden, dass kein unzulässiger Systemdruck insbesondere bei den Behältnissen (36/37) entsteht. Ebenso kann über den Druckaufnehmer (29) die Dichtheit des Systems und der Leitungen der angeschlossenen Behältnisse getestet werden.

Vorzugsweise erhalten die Rohstoffbehälter (36/37) einen Barcode zur Identifikation des Inhaltes, der Charge, der Dichte, Leitfähigkeit usw. Diese Angaben werden vor dem Mischvorgang eingelesen und mit den ermittelten Messergebnissen verglichen. Diese Dokumentation dient der Qualitätssicherung.

Das Behältnis (44a) wird über Anschluss (43), über die Saugleitung (46) und Ventil (45) zirkuliert. Vorzugsweise ist dieses Behältnis ein beutelartiger Kunststoffbehälter oder Folienbeutel.

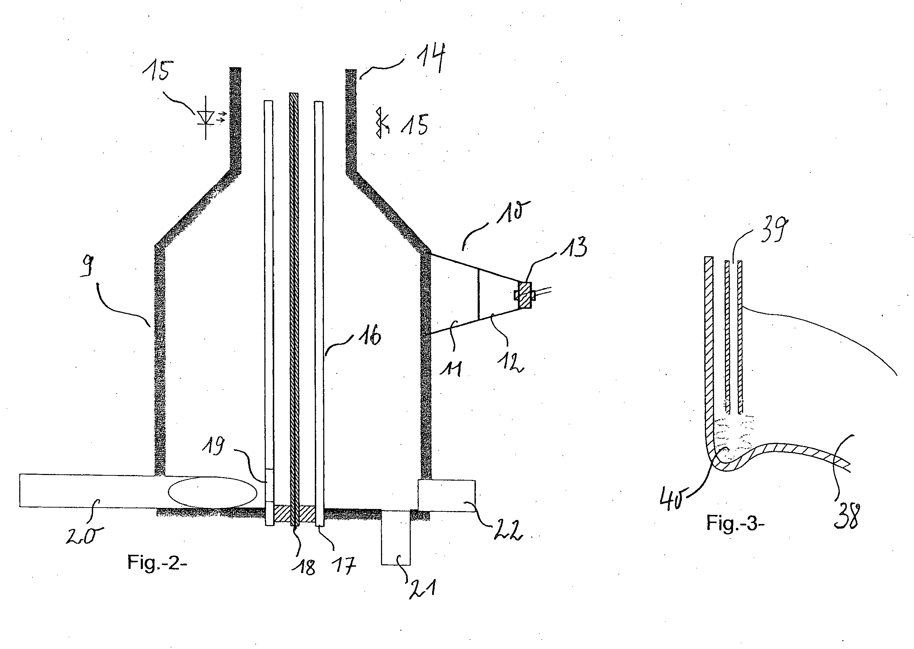

Der Rohstoffbehältern (36) wird vorzugsweise als Wegwerfeinheit geliefert, die Ausführung ist vorzugsweise ein Folienbeutel oder ein tankförmiges Gebilde gefüllt mit nassem, pulvrigem, granuliertem oder flüssigem Rohstoff. Bei einer tankförmigen Ausführung erfolgt die Zuführung in das Behältnis (36) wie Figur 3 zeigt, über die untere Anschlussleitung (33/39) und einen faltenbalgartigen Auslauf (40) der den Anschluss (39) fest auf den Tankboden drückt. Dabei ist der Tank nach innen gewölbt, sodass für die spätere Entleerung keine Restmengen zurückbleiben. Behältnis (37) ist ein wiederbefüllbarer Rohrstoffbehälter mit geradem, schrägem oder konischem Boden und einem unteren tangentialen Zulauf (77), der von vorne gesehen unten rechts mit einem großen Durchmesser z.B. 32 mm angeordnet ist.

Der Behälter weist einen oberen tangentialen Ablauf (78), der von vorne gesehen links oben angeordnet ist, und einen Durchmesser von z. B. 15 mm hat und einen weiteren unteren tangentialen Ablauf (76) der von vorne gesehen unten links angeordnet ist und einen Durchmesser von z. B. 20 mm aufweist. Das Behältnis (37) hat einen verschließbaren Deckel (75) mit Kegelspitze, die den Freiraum des Rotationsparaboloiden während des Mischvorganges im Behälter ausfüllt und dadurch eine sehr hohe Intensität der Vermischung liefert.

Der untere Abfluss (76) dient dem zusätzlichen Austrag des eingefüllten Rohstoffes bei geringen Durchflüssen durch den Behälter (37) zu Beginn des Mischvorganges, z.B. bei Verklumpung, und wird an der Außenwand des Behälters (37) so angeordnet, dass er am oberen Ende in den Ablauf (78) einmündet.

Nach fertiggestellter Mischung wird Ventil (41) geschlossen und Druckluft über den Druckluftsteueranschluss (68) eingeblasen. Dabei wird der Rohstoffbehälter (36/37) die Zirkulationsstrecke stromaufwärts über Ventil (45) einschließlich der Mischkammer (9) in das Behältnis (44/44a) entleert. Nach erfolgter Entleerung des Rohstoffbehälters (36/37) können die Anschlüsse (33/35) auf die Kurzschlussbrücke (34) zurückgesteckt werden, wobei mit Vorteil die Steckposition der Anschlussleitungen (35/33) mittels elektronischer Kontakte an der Kurzschlussbrücke (34) abgefragt werden. Die Abfrage der flexiblen Anschlussleitung lässt sich auch an den Kurzschlussbrücken (25/47) realisieren.

Zum Transfer wird das fertig gemischte Produkt aus Behältnis (44/44a)) über Pumpe (6) und Transferfilter (30) und Ventilen (31) zu den Verbrauchsstellen oder Lagertanks gefördert. Der Transfer bzw. die Entleerung kann sowohl über die Pumpe als auch bei Behälter (44a) mittels Lufteinspeisung am Anschluss (43) erfolgen.

Um nach der Mischung und dem Transfer hygienisch einwandfreie Verhältnisse wiederherzustellen, werden die Anschlüsse (33/35) auf die Kurzschlussbrücke (34) zurückgesteckt. Bei Verwendung des Behälters (44a) sind ebenfalls die Anschlüsse (43/46) auf Kurzschlussbrücke (47) zurückzustecken. Das System wird durch Öffnen des Ventiles (2) mit Wasser wieder gefüllt. Zur Entlüftung kann am oberen Ende des elektrolytischen Ozongenerators ein Entlüftungsventil (74) eingebaut werden, wobei die Lage des Entlüftungsventiles (74) nicht auf diesem Einbauort beschränkt ist. Durch Zirkulation der Flüssigkeit über Pumpe (6) und zuschalten der elektrolytischen Ozonzelle (32) werden dem System Sauerstoffradikale zur Dekontamination zugeführt. Dabei besteht die Ozonzelle aus zwei äußeren Kathoden und einer innenliegenden vorzugsweise aus graphithaltigem Material bestehenden ringförmigen Anode. Die im Kreislauf befindliche Flüssigkeit wird durch Öffnen des Ventiles (23) zum Abfluss (28) ausgespült.

### Figur 2

Alternativ zu der optoelektronischen Messung am oberen transparenten Ende der Mischkammer kann ein kapazitiver Füllstandsensor (16) eingesetzt werden, dessen Elektroden von einem äußeren Metallrohr (17) und einem koaxial darin angeordnetem isolierten Draht (18) oder Stab gebildet werden.

Das Rohr ist auf der einen Seite offen und hat mindestens eine seitliche Öffnung (19). Mit ansteigendem Füllstand wird der Innenraum zwischen Rohr und Stab mit Flüssigkeit aufgefüllt.

Insgesamt verhält sich ein solcher Sensor wie ein Kondensator, dessen Kapazität ausgehend von einem Anfangswert linear mit dem Füllstand zunimmt. Zur Signalgewinnung eignet sich wegen der Einfachheit z.B. eine Oszillatorschaltung, die eine Schwingung mit einer der Kapazität proportionalen Periodendauer erzeugt. Dieses Signal kann mit einem Mikroprozessor der Mischanlage leicht ausgewertet werden. Dabei können die Signale des kapazitiven Sensors (16) mit den Flussmesserwerten (3,4) mittels Rechner der Mischanlage ausgewertet bzw. verglichen werden.

Es hat sich gezeigt, dass mit diesem Verfahren auch eine Dichtemessung möglich ist.

Allerdings ist parallel zu der Kapazität des kapazitiven Sensors ein Verlustwiderstand wirksam, der von der Leitfähigkeit des Wassers abhängt. Dieser bleibt vernachlässigbar, wenn eine ausreichend hohe Messfrequenz benutzt wird.

Bei Flüssigkeiten mit höherer Leitfähigkeit kann dieser Verlustwiderstand trotz höheren Messfrequenzen zu erheblichen Messfehlern führen. Deshalb ist bei höheren Leitfähigkeiten diese Art der Dichtemessung nur eingeschränkt möglich.

Zur Lösung wird hier ein Ultraschallmessverfahren vorgeschlagen. Der Ultraschallsensor (10) kann dabei online zur Messung der spezifischen Dichte herangezogen werden. Der Messkörper (10) erhält einen einzigen Ultraschall-sensor (13) in Form einer Piezoscheibe zur Erzeugung einer Ultraschallwelle die auf die Oberfläche des zu messenden Fluids gerichtet ist und zum Empfang sowohl eines Referenzechos als auch eines Nutzechos dient. Dazu ist der Ultraschallsensor (10) in zwei Kegelschnittflächen (11,12) angeordnet. Die Grenzschicht zwischen diesen beiden Kegelschnittflächen dient zur Erzeugung des Referenzechos. Das Nutzecho wird durch die Fluidoberfläche gebildet. Zur Unterdrückung von Störechos und optimaler Einkopplung des Nutzechos in das Fluid, sind die Kegelschnittflächen (11) vorzugsweise aus Edelstahl und (12) aus Kunststoff gebildet. Das online ermittelte Dichte Messergebnis kann über einen Drucker neben Angabe der Leitfähigkeit und der Temperatur als Messwertprotokoll ausgedruckt werden.

Bei der Kapazitiven- wie auch der Ultraschalldichtemessung wird die Dichte dabei jeweils relativ zur Dichte des Reinstwassers ermittelt. Durch diese Messmethode kann die Dichtemesseung einfacher gestaltet werden, weil zunächst vor jedem Mischvorgang die Dichtemesseinheit mit der Dichte des Reinstwassers kalibriert wird, und bei der Online Messung der Erwartungswert der Fertiglösung als bekannt vorausgesetzt wird.

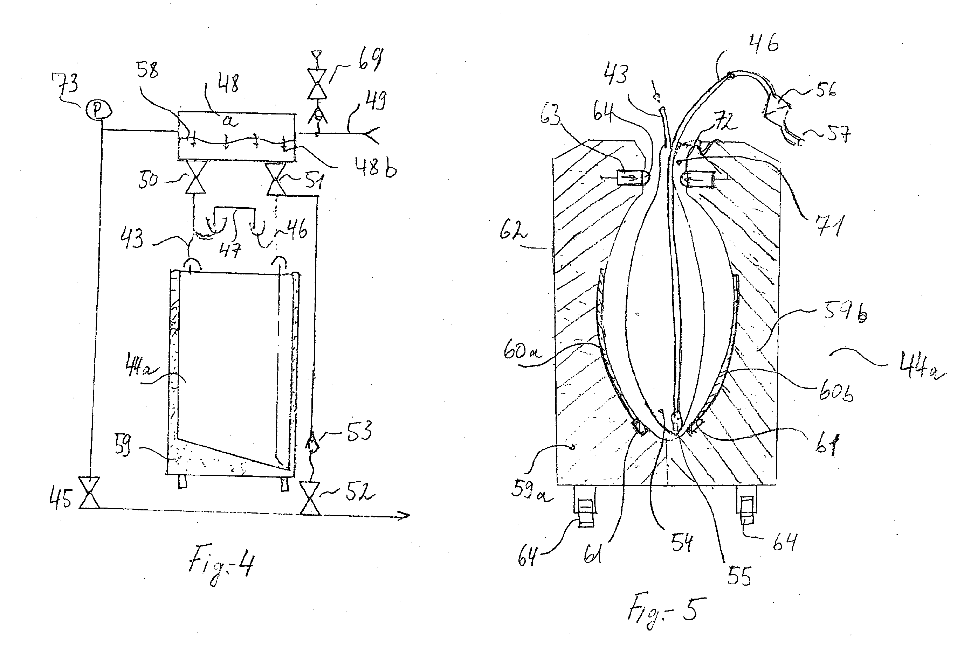

Figur 4 zeigt die Herstellung hochsteriler Lösung über einen Sterilfilter (48a/b) und ein vorzugsweise beutelartiges Behältnis (44a), das in einem isoliertem Schalenkörper (49) eingebettet ist. Die Befüllung des Behältnisses erfolgt wie bereits beschrieben über Ventil (45) und Filtermembran (58). Dabei kann wahlweise über Ventil (51) nach unten oder Ventil (50) von oben befüllt werden. Während des

Mischvorganges wird Permeat über die Zulaufleitung (49) über den Primärraum des Sterilfilters (48a) gefördert, wobei Pumpe (6) die Zirkulation übernimmt. Gleichzeitig wird ein kleiner Nebenstrom über Ventil (52/53) mitzirkuliert. Mit dieser Methode ist es möglich das Behältnis (44a) steril zu befüllen und trotzdem über das Behältnis (44a) zu zirkulieren. Ein weiterer Vorteil ist, dass der Primärraum des Sterilfilters (48a) permanent überströmt wird und ein Blockieren durch nicht gelöste Rohstoffe verhindert wird.

Zur Validierung des Sterilfilters (48) wird mittels Lufteintrag über Zuluft (69) die Flüssigkeit im Primärraum (48a) und durch das zunächst geöffnete Ventil (45) verdrängt. Bei geschlossenem Ventilen (45/41) und geöffneten Ventilen (51/52/23) wird bei laufender Pumpe (6) Unterdruck auf der Primärseite des Sterilfilters (48a) angelegt. Da der Filter luftundurchlässig ist, ist der über den Druckaufnehmer (73) ermittelte Druck ein Maß für die Filterdichtheit, weil bei einer Filterleckage ein schneller Druckabbau von Primär- zur Sekundärseite erfolgen würde. Der Filtertest ist auch mit Überdruck auf der Primärseite (48a) durchzuführen.

Figur 5 zeigt einen Beutelporter mit zwei isolierten Schalen (59a/b), die durch eine geeignete Konstruktion auseinanderklappbar sind. Der Porter steht auf Rollen (64). In einen oberen Schlitz (71) wird der Leerbeutel eingeschoben und an einem Befestigungshaken (72) eingerastet. Nach erfolgter Befüllung, Mischung und Temperierung der Lösung über die Leitungen (43/46) schmiegt sich die Beuteloberfläche den Konturen der isolierten Schalen (59a/b) so an, dass bei Bedarf die innenliegenden Heizmatten (60a/b) einen Temperaturverlust ausgleichen können. Um Ausfällungen des Rohstoffes im Beutel (54) zu vermeiden, können mit Vorteil Ultraschallsensoren (61) oder eine andere vibrationstechnische mechanische Beeinflussung der Flüssigkeit im Beutelporter (62) angebracht werden. Zum Nachheizen der gebrauchsfertigen Lösung besteht auch die Möglichkeit, die Anschlussleitung (46) des Folienbeutels (54) in eine Schlauchheizeinheit einzulegen, um während des Flüssigkeitstransportes zu temperieren.

Zum Transfer wird an den Anschluss (43) Druckluft mit einer erforderlichen Sicherheitseinrichtung in Form eines Druckreglers, Überdruckventil und Schnelldruckentlüftung am Beutelporter installiert. Während des Transfers werden die Dichtzylinder (63) mit den Dichtlippen (64) mechanisch oder elektrisch so verriegelt, dass eine Diskonnektion bzw. Ruptur des Behältnisses (54) verhindert wird. Durch das Einbringen steriler Luft über Anschluss (43) wird die gebrauchsfertige, erwärmte Flüssigkeit über Anschluss (46), Einmalsterilfilter (56) und Überleitsystem (57) zum Einsatzort gebracht. Mit Vorteil wird dabei der Einsatz eines hydrophoben Sterilfilters (56) vorgeschlagen. Damit wird verhindert, dass Luft über das Überleitsystem (57) zum Patienten gelangt. Anstelle des Lufteintrages in den Anschluss (43) könnte auch ein Doppelbeutel mit äußerer Umhüllung so eingesetzt werden, dass die äußere Beutelschicht zum Lufteintrag oder auch als Reservoir für gebrauchte Lösung genutzt werden könnten.

Nicht verbrauchte und nicht mehr benötigte fertiggemischte Lösung kann über die Anschlüsse (24/25/26/27/28) entleert werden. Die Entleerung erfolgt wie bereits für die Behältnisse (36/37) beschrieben. Luftdetektor (27) zeigt das Ende des Entleerungsvorganges an.

Alternativ zum Beutelporter (62) besteht auch die Möglichkeit, einen fahrbaren zylindrischen Glasbehälter einzusetzen, da Glas sowohl hygienisch einfach zu reinigen ist und thermisch gut isoliert. Es besteht auch die Möglichkeit, den Rohstoff direkt in den Beutel Fertiglösung (54) abzufüllen und so ohne Disposables (38) eine gebrauchsfertige Lösung herzustellen.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar.

### Legende

| | |
|---|---|
| 1. | Reinstwasserzulaufleitung |
| 2. | Wassereingangsventil |
| 3. | Flussmesser 1 |
| 4. | Flussmesser 2 |
| 5. | Rückschlagventil |
| 6. | Zirkulationspumpe (Drehzahlgeregelt oder Druckgesteuert) |
| 7. | Heizung |
| 8. | Leitfähigkeitsmessung mit Temperaturüberwachung |
| 9. | Messkammer |
| 10. | Ultraschalldichtesensor |
| 11. | Material 2 |
| 12. | Material 1 |
| 13. | Piezoscheibe |
| 14. | Transparente Messstrecke |
| 15. | Elektronische Messeinrichtung |
| 16. | Kapazitiver Füllstandsensor |
| 17. | Rohrförmige Aussenelektrode |
| 18. | Innenelektrode mit Isolierung |
| 19. | Seitliche Zuflussöffnung des kapazitiven Sensors |
| 20. | Tangentialer Einlauf |
| 21. | Abflussbohrung für Trenage |
| 22. | Transferleitung |
| 23. | Drainageventil/Verwurfventil |
| 24. | Obere Entleerungsanschluss |
| 25. | Kurzschlussbrücke Entleerung |
| 26. | Untere Entleerungsanschluss |
| 27. | Leererkennungssensor |
| 28. | Trenage |
| 29. | Druckaufnehmer |
| 30. | Transferfilter |
| 31. | Transferventil |
| 32. | Elektrolytische Ozonzelle |
| 33. | Anschluss untere Zuflussleitung Rohstoffbehälter |
| 34. | Kurzschlussbrücke |
| 35. | Anschluss obere Zuflussleitung Rohstoffbehälter |
| 36. | Mobile Rohstoffbehälter (Wegwerfbehälter) |
| 37. | Stationärer Rohstoffbehälter mit Deckel und unteren tangetialen Einlauf |
| 38. | Sichtausschnitt des Salzbehälters |
| 39. | Untere Zuflussleitung |
| 40. | Faltenbalgartiger Auslauf |
| 41. | Entleersperreventil Rohstoffbehälter |
| 42. | Statischer Mischer |
| 43. | Oberer Zulaufanschluss Behältnis Fertiglösung |
| 44. | /a Tank-/Behältnis-/Folienbeutel für Fertiglösung |
| 45. | Zirkulations- und Füllventil |
| 46. | Sauganschluss Fertiglösungsbehälter |
| 47. | Kurzschlussbrücke Fertiglösungsbehälter |
| 48. | Sterilfilter (a Primärseite, b Sekundärseite) |
| 49. | Zulaufleitung zu den Fertiglösungsbehälter |
| 50. | Füllventil Fertiglösungsbehälter |
| 51. | Mischventil Fertiglösungsbehälter |
| 52. | Zirkulationsventil Fertiglösungsbehälter |
| 53. | RS-Ventil Zirkulation Fertiglösung |
| 54. | Beutel Fertiglösung |
| 55. | Saug- und Ausströmanschluss |
| 56. | Einmalsterilfilter |
| 57. | Überleitsystem zum Patienten |
| 58. | Filtermembran |
| 59. | Isolierte Schalenkörper |
| 60. | Heizmattenauskleidung |
| 61. | Ultraschall- bzw. Vibrationsaktoren |
| 62. | Beutelporter bzw. Porter für Fertiglösung |
| 63. | Dichtzelinder |
| 64. | Dichtlippen |
| 65. | Beruhigungsstrecke |
| 66. | Konstantflussdrossel |
| 67. | Druckluftsteueranschluss |
| 68. | Druckluftsteueranschluss |
| 69. | Druckluftsteueranschluss |
| 70. | Tankentlüftung |
| 71. | Oberer Schlitz Beutelporter |
| 72. | Befestigungshaken Beutelporter |
| 73. | Druckaufnehmer Sterilfilter |
| 74. | Entlüftungsventil |
| 75. | Deckel mit Kegelspitze |
| 76. | Ablauf unten tangential kleinerer Durchmesser von vorne unten links |
| 77. | Zulauf unten tangential großer Durchmesser von vorne unten rechts |
| 78. | Ablauf oben tangential kleinster Durchmesser von vorne links oben |
| 79. | Probeentnahmeventil |

## Patentansprüche

1. Beutelporter für Fertiglösung,
**gekennzeichnet durch** zwei isolierte Schalen (59a, 59b), die auseinander klappbar sind und einen oberen Schlitz (71) aufweisen, **durch** den ein leerer Beutel (54) einschiebbar ist, der einen Zulaufanschluss (43) aufweist, **durch** den die Fertiglösung in den Beutel (54) einfüllbar ist, wonach sich der Beutel (54) an die Innenkonturen der Schalen (59a, 59b) anlegt, die mit innen liegenden Heizmatten (60a, 60b) versehen sind, die einen Temperaturverlust ausgleichen können,
wobei der Beutel (54) eine Anschlussleitung (46) mit einem Überleitungssystem (57) zum Patienten aufweist.

2. Beutelporter nach Anspruch 1,
**dadurch gekennzeichnet,**
**dass** an dem Schlitz (71) ein Befestigungshaken (72) angeordnet ist, in den der Beutel (54) einrasten kann.

3. Beutelporter nach den Ansprüchen 1 oder 2,
**dadurch gekennzeichnet,**
**dass** an dem Schlitz (71) Dichtzylinder (63) mit Dichtlippen (64) zur mechanischen oder elektrischen Verriegelung angeordnet sind.

4. Beutelporter nach den Ansprüchen 1 bis 3,
**dadurch gekennzeichnet,**
**dass** in den Schalen (59a, 59b) Ultraschall- oder Vibrationsaktoren (61) zum Einwirken auf die Fertiglösung in dem Beutel (54) angeordnet sind.

5. Beutelporter nach den Ansprüchen 1 bis 4,
**dadurch gekennzeichnet,**
**dass** sterile Luft durch den Zulaufanschluss (43) in den Beutel (54) zur Abgabe der Fertiglösung durch die Anschlussleitung (46) einbringbar ist.

6. Beutelporter nach den Ansprüchen 1 bis 4,
**dadurch gekennzeichnet,**
**dass** der Beutel ein Doppelbeutel ist mit einer äußeren Umhüllung, die zum Lufteintrag nutzbar ist.

7. Beutelporter nach den Ansprüchen 1 bis 8,
**dadurch gekennzeichnet,**
**dass** die Anschlussleitung (56) mit einem Einmalsterilfilter (56) versehen ist.

8. Beutelporter nach Anspruch 7,
**dadurch gekennzeichnet,**
**dass** der Filter luftundurchlässig ist.

9. Beutelporter nach den Ansprüchen 1 bis 8,
**dadurch gekennzeichnet,**
**dass** der Beutelporter auf Rollen (64) steht.

10. Beutelporter nach den Ansprüchen 1 bis 9,
**dadurch gekennzeichnet,**
**dass** der Beutel (54) ein Folienbeutel ist.
